(19) **Europäisches Patentamt / European Patent Office / Office européen des brevets**

(11) **EP 3 443 907 A1**

(12) **EUROPEAN PATENT APPLICATION**
published in accordance with Art. 153(4) EPC

(43) Date of publication:
**20.02.2019 Bulletin 2019/08**

(21) Application number: **17782075.0**

(22) Date of filing: **18.01.2017**

(51) Int Cl.:
**A61B 6/03** *(2006.01)*        **G01T 1/161** *(2006.01)*

(86) International application number:
**PCT/JP2017/001460**

(87) International publication number:
**WO 2017/179256 (19.10.2017 Gazette 2017/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.04.2016 JP 2016080087**

(71) Applicant: **Nihon Medi-Physics Co., Ltd.**
**Tokyo 136-0075 (JP)**

(72) Inventors:
• **HAMADA, Kazuo**
  **Tokyo 136-0075 (JP)**
• **NISHIDA, Kazumasa**
  **Tokyo 136-0075 (JP)**
• **DAISAKI, Hiromitsu**
  **Tokyo 136-0075 (JP)**

(74) Representative: **TBK**
**Bavariaring 4-6**
**80336 München (DE)**

(54) **AUTOMATIC REMOVAL OF PHYSIOLOGICAL ACCUMULATIONS FROM NUCLEAR MEDICINE IMAGE, AND AUTOMATIC SEGMENTATION OF CT IMAGE**

(57)    Disclosed is a method for automatically removing physiological accumulation from a nuclear medical image. Also disclosed as a preferred application example of this method is a method for performing automatic segmentation of a CT image that can be used to automatically remove physiological accumulation from a nuclear medical image. In an exemplary embodiment, a histogram of pixel values is created based on a CT image, and a boundary between an air region and a fat region, a boundary between the fat region and a soft tissue region, and a boundary between the soft tissue region and a bone region are determined. Based on this result, a head region and a lower abdomen region in the CT image are automatically specified. Based on this result, a head region and a bladder region are specified in a nuclear medical image, and a physiological high accumulation region is specified. A part corresponding to the physiological high accumulation region is masked to display the nuclear medical image.

Fig. 2

## Description

### Field

The present application relates to automatic removal of physiological accumulation from a nuclear medical image and automatic segmentation of a CT image.

### Background

Nuclear medical technology is the technology of imaging how radioactive medicines administered into the body gather at an organ or a body tissue. This technology can be used for obtaining functional changes in blood flow, metabolism, and the like as image information, thereby providing information useful for disease diagnosis, identification of disease stage and prognosis, and determination of therapeutic effect.

Paragraphs 0027 and 0029 in Japanese Patent Application Laid-open No. 2013-088386 indicate that position information on a thigh bone extracted from a CT image is used for extracting only a portion corresponding to the thigh bone from a PET image.

### List of References

### Patent Literature

Patent Literature 1: Japanese Patent Application Laid-open No. 2013-088386

### Summary of the Invention

Some radioactive medicines, however, accumulate in a region where blood flow or metabolism is naturally high, such as the brain and the bladder, irrespective of whether the region suffers from a disorder. Such physiological accumulation is displayed bright on a nuclear medical image, which may hinder image diagnosis.

The present application discloses a method for automatically removing physiological accumulation from a nuclear medical image. The present application also discloses a method for performing automatic segmentation of a CT image that can be preferably applied to automatic removal of physiological accumulation from a nuclear medical image.

A first aspect of the present invention provides a method for automatically classifying pixel values in a CT image. This aspect of the invention implements processing including:

- creating a histogram of pixel values based on the CT image;

- determining a fat region peak that is a peak in a fat region in the histogram;

- determining a soft region peak that is a peak in a soft tissue region in the histogram;

- determining a first threshold that is a threshold representing an upper limit of bin values in an air region, based on a frequency value of the fat region peak in a region of bin values smaller than a bin value of the fat region peak;

- determining a second threshold that is a threshold representing a bin value of a boundary between the fat region and the soft tissue region; and

- determining a third threshold that is a threshold representing a lower limit of bin values in a bone region, based on a frequency value of the soft region peak in a region of bin values larger than a bin value corresponding to the soft region peak.

According to the first aspect of the present invention, pixel values in a CT image can be classified into pixel values in an air region, pixel values in a fat region, pixel values in a soft tissue region, and pixel values in a bone region, and hence a CT image can be flexibly displayed depending on the intended purpose. For example, only the soft tissue region can be highlighted and displayed, or only the bone region can be highlighted and displayed. The first aspect of the present invention provides also very useful for automatically segmenting regions in a CT image. For example, a region having a large number of pixels with pixel values corresponding to the air region is a lung field with high possibility. One region having a large number of pixels with pixel values corresponding to the bone region is, for example, a pelvis with high possibility. In this manner, the use of the results of the pixel value classification according to the first aspect of the present invention enables the region in a CT image to be automatically segmented.

In some embodiments, the creating of a histogram may include:

- creating a reference image that is a binary image from which a bed portion is removed to leave only a human body portion in the CT image; and

- creating the histogram by using only pixels where data is present in corresponding pixels in the reference image among pixels in the CT image.

Analysis is performed with a CT image from which a bed portion, which is not related to the human body, has been removed, and hence the adequateness of analysis results improves.

In some embodiments, the histogram may be subjected to smoothing processing before the fat region peak and the soft region peak are determined.

In some embodiments,

- the determining of a fat region peak may include determining a maximum frequency value in a range from a lower limit bin value that is set so as to include the fat region peak to a reference bin value that is a bin value corresponding to water, and when the maximum frequency value is not a frequency value of the reference bin value, determining that the maximum frequency value and the bin value corresponding to the maximum frequency value are respectively a frequency value and a bin value of the fat region peak, and

- the processing may further include determining the second threshold to be a bin value with which a frequency value is minimum in a range from the bin value of the fat region peak to the reference bin value.

When the maximum frequency value in the range from the lower limit bin value to the reference bin value is a frequency value of the reference bin value, in some embodiments, the determining of a fat region peak may include:

- determining the second threshold to be a bin value with which a change amount of a frequency value is minimum in the range from the lower limit bin value to the reference bin value, the change amount being a value defined as:

$$\text{Difference of bin value i from previous bin value} = (\text{Frequency value of bin value i-1}) - (\text{Frequency value of bin value i});$$

$$\text{Difference of bin value i from subsequent bin value} = (\text{Frequency value of bin value i}) - (\text{Frequency value of bin value i+1});$$

and

$$\text{Change amount of bin value i} = (\text{Difference of bin value i from previous bin value})^2 + (\text{Difference of bin value i from subsequent bin value})^2$$

where i is an integer;
- determining an examination bin value to be either of:
- a bin value having the maximum change amount in the range from the lower limit bin value to the second threshold; and
- a bin value corresponding to a frequency value that first falls below a frequency value of the second threshold among frequency values varying from the second threshold toward the lower limit bin value; and
- determining that a bin value with which a value of the difference from the previous bin value is positive and minimum in the range from an examination boundary value to the second threshold and the frequency value corresponding to the bin value are respectively a bin value and a frequency value of the fat region peak.

When a bin value with which the value of the difference from the previous bin value is not found in the range from the examination boundary value to the second threshold, in some embodiments, the determining of a fat region peak may include determining that a maximum frequency value in the range from the lower limit bin value to the second threshold and the bin value corresponding to the maximum frequency value are respectively a frequency value and a bin value of the fat region peak.

The fat region peak can be determined by the series of processing described above in most cases.

In some embodiments, the determining of a soft region peak may include performing peak detection processing within a predetermined bin value range.

In some embodiments, the first threshold may be determined to be either of:

- a bin value that has a frequency value equal to or smaller than a predetermined proportion of a frequency value of the fat region peak or smaller than the predetermined proportion in the range from the lower limit bin value to the second threshold; and

- a bin value having the maximum change amount in the range from the lower limit bin value to the bin value of the fat region peak.

In some embodiments, the third threshold may be determined to be a bin value that is larger than a bin value corresponding to the soft region peak and has a frequency value accounting for a predetermined proportion of a frequency value of the soft region peak.

The first to third thresholds can be determined by the processing described above in most cases.

A second aspect of the present invention provides a method for automatically segmenting a region of the CT image for which pixel values are classified according to the first aspect of the present invention and the first to third thresholds are determined. This aspect of the invention implements processing including:

- creating an air region volume graph that is a graph having one axis representing slice numbers of axial section slices in the CT image and another axis representing the volume of at least a part of a pixel group with pixel values equal to or smaller than the first threshold or smaller than the first threshold in slices corresponding to the slice numbers; and

- determining an axial section slice that is closer to a vertex than an axial section slice for which the air region volume graph exhibits a maximum value, the axial section slice having a volume value accounting for a predetermined proportion of the maximum value of the air region volume graph, to be a breast start slice located at an upper end of a breast.

According to the second aspect of the present invention, a breast start slice located at the upper end of the breast in axial section slices in the CT image can be automatically determined. Thus, for example, the position of the body can be easily grasped in the CT image. When there is a nuclear medical image registered with a CT image, the breast start slice position can be easily grasped in this nuclear medical image.

In some embodiments, the processing may include subjecting the air region volume graph to smoothing processing before determining the breast start slice.

In some embodiments, the processing may include:

- creating a soft region volume graph that is a graph having one axis representing slice numbers of axial section slices in the CT image and another axis representing the volume of a pixel group with pixel values between the second threshold and the third threshold in slices corresponding to the slice numbers; and

- determining an axial section slice that is closer to a lower extremity than an axial section slice for which the air region volume graph exhibits a maximum value, the axial section slice having the largest volume value of the soft region volume graph, to be an upper abdomen start slice located at an upper end of an upper abdomen.

In some embodiments, the processing may include subjecting the soft region volume graph to smoothing processing before determining the upper abdomen start slice.

In some embodiments, the processing may include determining an axial section slice that is closer to a vertex than the breast start slice and has the largest volume value of the air region volume graph to be a neck start slice located at an upper end of a neck.

In some embodiments, the processing may include:

- performing 3D labeling on axial section slices closer to the vertex than the neck start slice;

- determining a head label that is a label located at a center in the axial section slice; and

- calculating, for axial section slices from the neck start slice to the vertex side, the volume of a pixel group that is located in a region corresponding to the head label and has pixel values between the second threshold and the third threshold, and determining an axial section slice having the volume that first becomes zero to be a head start slice located at an upper end of a head.

In some embodiments, the processing may further include determining, for axial section slices closer to the lower extremity than the upper abdomen start slice, a lower abdomen start slice located at an upper end of a lower abdomen based on a change in the volume of a bone.

In some embodiments, the determining of a lower abdomen start slice may include:

- performing 3D labeling on axial section slices closer to the lower extremity than the upper abdomen start slice, and determining a trunk label, which is the largest label;

- extracting, for the axial section slices close to the lower extremity than the upper abdomen start slice, a pixel group that is located in a region corresponding to the trunk label and has pixel values equal to or larger than the third threshold or larger than the third threshold, and performing 3D labeling on the extracted pixel group to determine a backbone/pelvis/thigh bone label, which is the largest label;

- creating, for axial section slices closer to the lower extremity than the upper abdomen start slice, a bounding rectangle of a pixel group that corresponds to the backbone/pelvis/thigh bone label and has pixel values equal to or larger than the third threshold or larger than the third threshold; and

- determining an axial section slice having the largest change amount of the bounding rectangle to be the lower abdomen start slice located at the upper end of the lower abdomen.

In some embodiments, the processing may further include:

performing, for axial section slices closer to the lower extremity than the lower abdomen start slice, the following:

- labeling a pixel group that is located in a region corresponding to the trunk label and has pixel values equal to or larger than the third threshold or larger than the third threshold;

- extracting two labels having largest and second largest sizes in the same slice, and when the two labels do not overlap with each other, determining the two labels to be thigh bone labels;

- extracting, when the thigh bone labels are successfully determined, a hole for each of the thigh bone labels; and

- calculating, when the hole is successfully extracted, a thigh bone volume that is the volume of a pixel group that is located in a region corresponding to the thigh bone label and has pixel values equal to or larger than the third threshold or larger than the third threshold, and

the processing may further include:

- calculating a maximum value of the volume of a pixel group that is located in a region corresponding to the trunk label and has pixel values equal to or larger than the third threshold or larger than the third threshold in the axial section slices closer to the lower extremity than the lower abdomen start slice; and

- determining a slice having a value of the thigh bone volume accounting for a predetermined proportion of the maximum value first out of the axial section slices for which the thigh bone volume is successfully calculated among the axial section slices closer to the lower extremity than the lower abdomen start slice to be a lower extremity start slice located at an upper end of the lower extremity.

The above-described embodiments enable, for example, determination on where a slice located at an upper end of the breast, a slice located at an upper end of the upper abdomen, a slice located at an upper end of the neck, a slice located at an upper end of the head, a slice located at an upper end of the lower abdomen, and a slice located at an upper end of the lower extremity are located in axial section slices in a CT image. Thus, for example, by displaying the slice positions, the position of the body in the CT image can be easily grasped. When there is a nuclear medical image registered with a CT image, the position of the body can be easily grasped in this nuclear medical image.

A third aspect of the present invention provides a method for automatically removing physiological accumulation from a nuclear medical image. This aspect of the invention uses information on the slice position of the head start slice and information on the slice position of the neck start slice. These slices are determined according to an embodiment of the second aspect of the present invention. This aspect of the invention implements processing including:

- setting a maximum pixel value search region in the nuclear medical image by using the slice position of the head start slice and the slice position of the neck start slice;

- determining a pixel having a maximum pixel value in the maximum pixel value search region; and

- determining a high accumulation region in the head by an approach of region growing from the pixel having the maximum pixel value.

According to the third aspect of the present invention, a high accumulation region in the head can be automatically determined from a nuclear medical image, and hence other lesions in the nuclear medical image can be easily observed. For example, by displaying the nuclear medical image while the high accumulation region is masked, the nuclear medical image can be observed without being interfered by unnecessary signals from the head.

A fourth aspect of the present invention provides another method for automatically removing physiological accumulation from a nuclear medical image. This aspect of the invention uses information on the slice position of the lower abdomen start slice and information on the slice position of the lower extremity start slice. These slices are determined according to an embodiment of the second aspect of the present invention. This aspect of the invention implements processing including:

- setting a maximum pixel value search region in the nuclear medical image by using the slice position of the lower abdomen start slice and the slice position of the lower extremity start slice;

- determining a pixel having a maximum pixel value in the maximum pixel value search region; and

- determining a high accumulation region in a bladder by an approach of region growing from the pixel having the maximum pixel value.

According to the fourth aspect of the present invention, a high accumulation region in the bladder is automatically determined from a nuclear medical image, and hence the nuclear medical image can be easily observed. For example, by displaying the nuclear medical image while the high accumulation region is masked, the nuclear medical image can be observed without being interfered by unnecessary signals from the bladder.

An embodiment of the present invention provides a computer program including a program instruction configured to cause a device to implement the processing described above when executed by processing means in the device. An embodiment of the present invention provides a method to be implemented by a device when processing means in the device executes a program instruction, and the method includes implementing the processing described above.

An embodiment of the present invention provides a device including processing means and storage means having a program instruction stored thereon, and the program instruction is configured to cause the device to implement the processing described above when executed by the processing means.

Some embodiments of the invention in the present application that are currently considered preferable are specified in the appended claims. However, the configurations specified in the appended claims do not necessarily include all the novel technical concept disclosed herein and in the accompanying drawings. It should be noted that the applicant claims the right to have a patent granted on all the novel technical concept disclosed herein and in the accompanying drawings regardless of whether they are specified in the current set of the appended claims.

**Brief Description of Drawings**

FIG. 1 is a diagram for describing a hardware configuration of a system that can embody the present invention.

FIG. 2 is a flowchart for describing the procedure of processing 200, which is an example of novel processing disclosed herein, for automatically segmenting a CT image and automatically removing physiological accumulation from a nuclear medical image.

FIG. 3 is a flowchart for describing a specific example of processing in Step 212 in FIG. 2.

FIG. 4 includes diagrams for describing bed portion removal processing in Step 306.

FIG. 5 is an example of a histogram created in Step 308.

FIG. 6A is a flowchart for describing processing 600, which is a specific example of processing in Step 310 in FIG. 3.

FIG. 6B is a diagram for describing an examination bin value determined in Step 614.

FIG. 7 is a diagram schematically representing the relations among the histogram, a fat region peak, a soft region peak, and first to third thresholds.

FIG. 8 is a flowchart for describing processing 800, which is a specific example of processing in Step 214 in FIG. 2.

FIG. 9 is a display example of a graph created in Step 806.

FIG. 10 is a diagram illustrating the positions of a breast start slice, an upper abdomen start slice, and a neck start slice determined in an example of Steps 808 to 812 on the graphs in FIG. 9.

FIG. 11 is a diagram for describing processing in Step 814.

FIG. 12 is a flowchart for describing processing 1200, which is a specific example of Step 816 in FIG. 8.

FIG. 13 is a diagram for describing processing in Step 1204.

FIG. 14A is a diagram for describing processing in Step 1206.

FIG. 14B is an example in which a bounding rectangle is superimposed and displayed on a backbone/pelvis/thigh bone label in FIG. 14A.

FIG. 15 is a flowchart for describing processing 1500, which is a specific example of Step 818 in FIG. 8.

FIG. 16 is a diagram for describing processing in Step 1508.

FIG. 17 is a flowchart for describing processing 1700, which is a specific example of processing in Step 216 in FIG. 2.

FIG. 18 illustrates a display example of results.

**Description of Exemplary Examples**

Referring to the accompanying drawings, exemplary embodiments of the technical concept disclosed herein are described below.

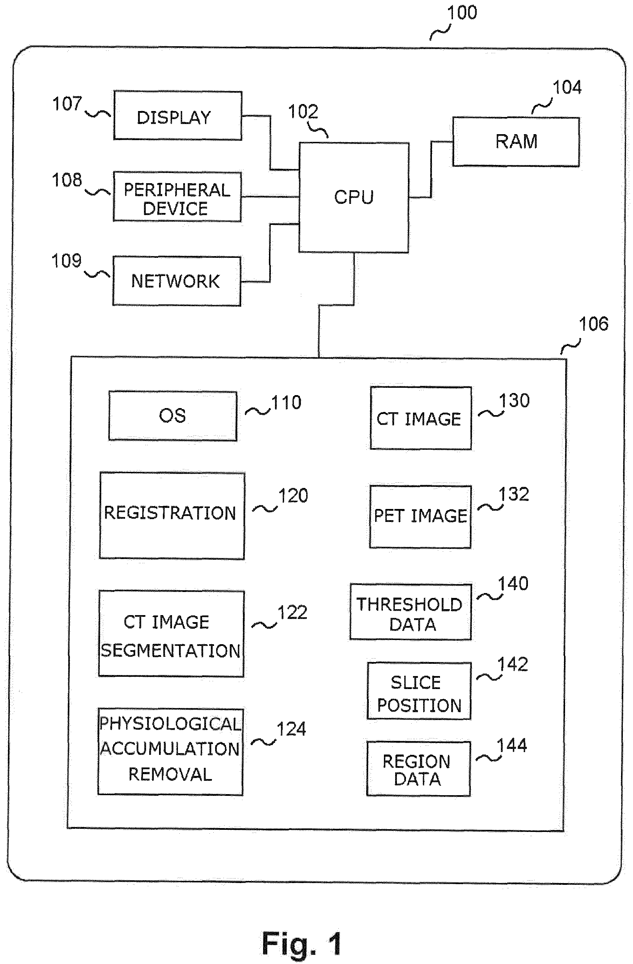

FIG. 1 is a diagram for describing the hardware configuration of a system 100 that can embody the present invention. As illustrated in FIG. 1, the system 100 is similar to a commonly-used computer in terms of hardware, and can include a central processing unit (CPU) 102, a main storage device 104, a mass storage device 106, a display interface 107, a peripheral device interface 108, and a network interface 109. Similarly to a commonly-used computer, a high-speed random access memory (RAM) can be used as the main storage device 104, and an inexpensive large-capacity hard disk or solid state drive (SSD) can be used as the mass storage device 106. A display for displaying information can be connected to the system 100. The display is connected through the display interface 107. A user interface such as a keyboard, a mouse, and a touch panel can be connected to the system 100. The user interface is connected through the peripheral device interface 108. The network interface 109 can be used to connect the system 100 to another computer or the Internet through a network.
In the mass storage device 106, an operating system (OS) 110, a registration program 120, a CT image segmentation program 122, and a physiological accumulation removal program 124 are stored. The most basic functions of the system 100 are provided by the OS 110 executed by the CPU 102. The registration program 120 is a computer program for performing alignment (registration) between a CT image and a nuclear medical image. The CT image segmentation program 122 includes program instructions related to novel processing disclosed herein. When the CPU 102 executes at least a part of the instructions, the system 100 can implement the segmentation of a CT image, that is, automatic segmentation of a body region. The physiological accumulation removal program 124 also includes program instructions related to novel processing disclosed herein. When the CPU 102 executes at least a part of the instructions, the system 100 can automatically remove physiological accumulation from a nuclear medical image.

In the mass storage device 106, CT images 130 and nuclear medical images 132 can be further stored. A CT image 130 is three-dimensional image data in which each pixel value corresponds to a CT value, and is image data to be analyzed or operated by the CT image segmentation program 122. A nuclear medical image 132 is a PET image in the present example, and is three-dimensional image data in which each pixel value corresponds to a radiation count value. The nuclear medical image 132 is subjected to analysis or operation by the physiological accumulation removal program 124.

In the mass storage device 106, pieces of data 140, 142, and 144 may be further stored. These pieces of data are described later when the pieces of data are generated.

Other than the components illustrated in FIG. 1, the system 100 may include the same configuration as that of a device included in a general computer system, such as a power source and a cooling device. Various embodiments of a computer system employing various techniques have been known, such as distributed, redundant, or virtualized storage devices, use of a plurality of CPUs, CPU virtualization, use of a processor (such as a DSP) dedicated to determination processing, and implementation of determination processing in hardware to be used in combination with a CPU. The invention disclosed herein may be installed on any type of computer systems, and the scope of the invention is not limited by the type of computer systems. The technical concept disclosed in the specification can be generally embodied by (1) a computer program including an instruction configured to cause, when executed by processing means, a device or a system including the processing means to implement various kinds of processing described in the specification, (2) an operation method for a device or a system implemented by the processing means executing the computer program, and (3) a device or a system including the computer program and processing means configured to execute the program. As described above, a part of the software processing may be implemented in hardware.

Note that the CT image 130, a PET image 132, and various kinds of data 140, 142, and 144 are not stored in the mass storage device 106 in many cases at the time of manufacture, sales, and start-up of the system 100. The CT image 130 and the PET image 132 may be, for example, data transferred from an external device to the system 100 through the peripheral device interface 108 or the network interface 109. The data 140, 142, and 144 may be data stored in the mass storage device 106 as a result of the execution of at least a part of the CT image segmentation program 122 and the physiological accumulation removal program 124 by the CPU 102. It should be understood that the scope of the invention disclosed herein is not limited to whether image data is stored in a storage device.

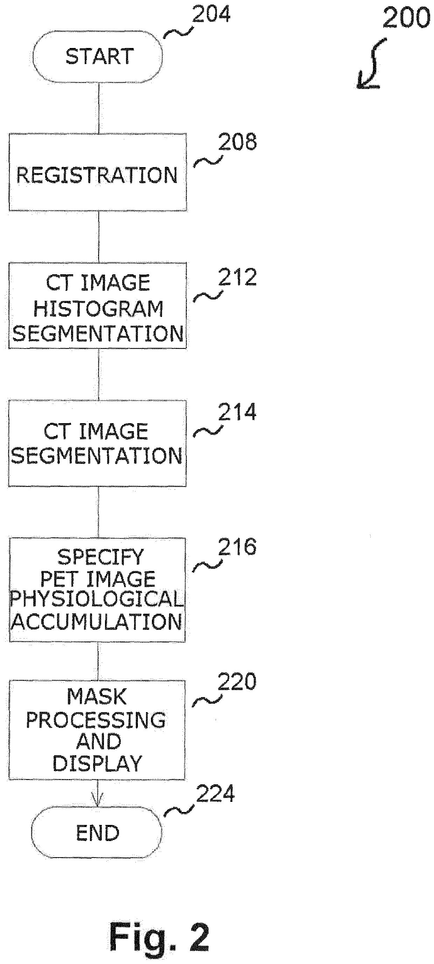

Next, the procedure of processing 200, which is an example of novel processing disclosed herein, for automatically segmenting a CT image and automatically removing physiological accumulation from a nuclear medical image is described with reference to FIG. 2. For example, the processing 200 may be implemented by the system 100 when the CPU 102 executes the CT image segmentation program 122 or the physiological accumulation removal program 124.

Step 204 represents the start of the processing. In Step 208, alignment (registration) between the nuclear medical image 132 and the CT image 130 is performed. Specifically, the orientation, size, and position of the body are three-dimensionally aligned between the CT image 130 and the nuclear medical image 132. This processing enables the CT image 130 and the nuclear medical image 132 to be compared with each other. The registration processing may be implemented by the system 100 when the CPU 102 executes the registration program 120.

The registration function is included in many commercially available nuclear medical image data analysis programs, and hence in the present example, the registration may be performed by existing methods implemented in such programs or other publicly known methods. The registration may be performed by a manual method. Specifically, a CT image and a nuclear medical image may be both displayed as images, and one image may be translated or rotated to be aligned with the other image by operation with a mouse.

In the following description, it should be understood that the pieces of image data 130 and 132 have been registered. In some embodiments, registration between the pieces of image data 130 and 132 may already be completed. For example, when pieces of image data 130 and 132 are simultaneously taken by a device in which SPECT or PET and CT are integrated, registration between the CT image 130 and the nuclear medical image 132 may already be completed when the images are output from the device. In this case, the processing in Step 208 is unnecessary. In such embodiments, the registration program 120 is also unnecessary.

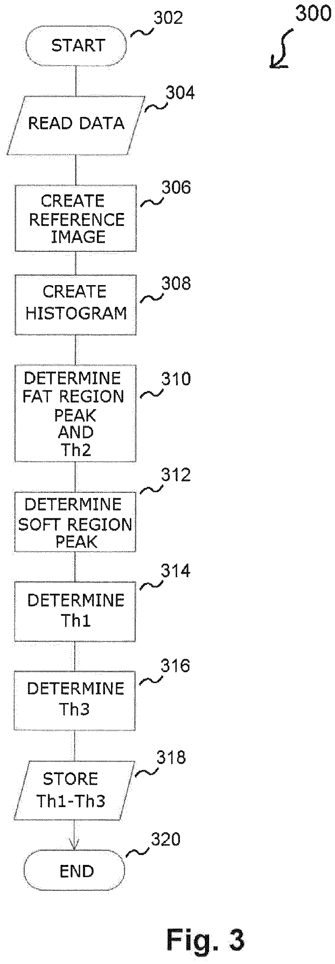

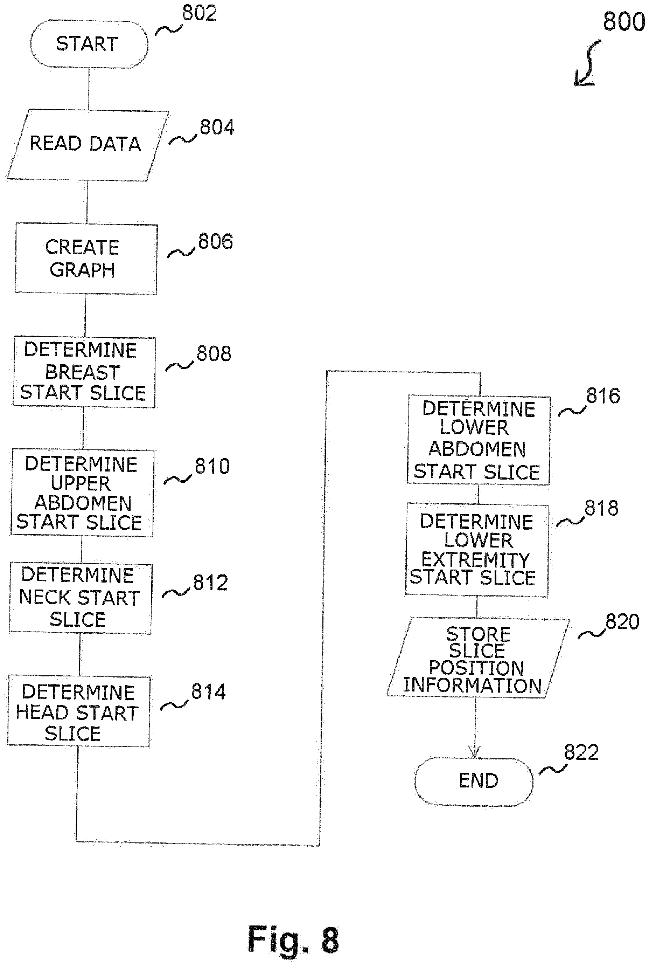

In Step 212, as post-processing for Step 214, the processing 200 segments pixel values in the CT image 130 into an air region, a fat region, a soft tissue region, and a bone region. An example of the processing in Step 212 is described in detail later with reference to FIG. 3. In Step 214, the processing 200 uses the result in Step 212 to specify sites of the body in the CT image 130. Specifically, the processing 200 determines a slice located at a specific site among axial section slices in the CT image 130. For example, the processing 200 can determine which of the axial section slices is a slice located at an upper end of the breast, a slice located at an upper end of the upper abdomen, a slice located at an upper end of the neck, a slice located at an upper end of the head, a slice located at an upper end of the lower abdomen, or a slice located at an upper end of the lower extremity. An example of the processing in Step 214 is described in detail later with reference to FIGS. 8 to 16.

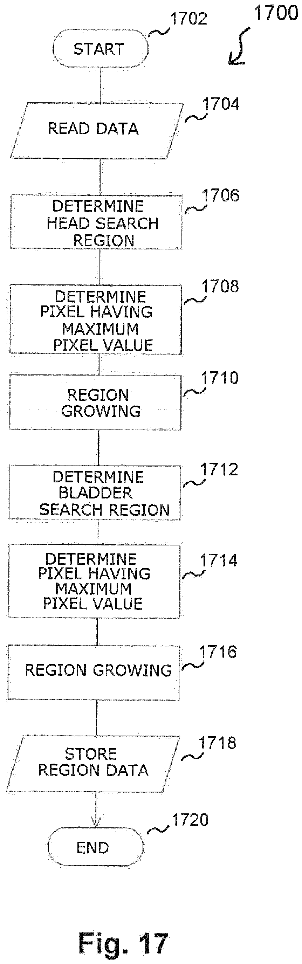

In Step 216, the processing 200 specifies physiological accumulation in the nuclear medical image 132 based on the result in Step 212. An example of the processing in Step 216 is described in detail later with reference to FIG. 17.

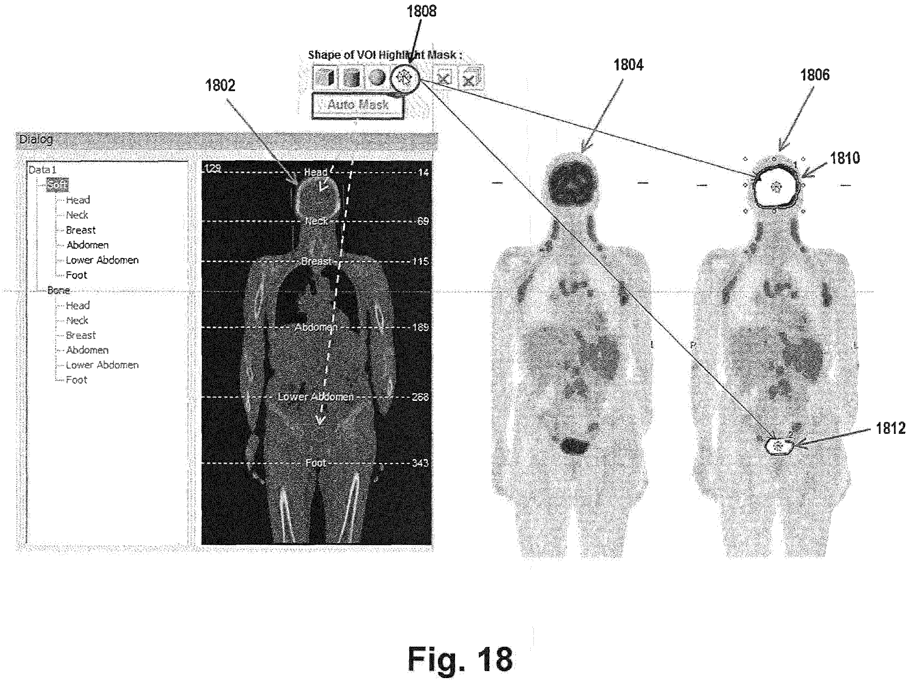

In Step 220, the processing 200 displays the nuclear medical image 132 by masking a part where the physiological accumulation is present based on the result in Step 216. FIG. 18 illustrates a display example.

Step 224 represents the end of the processing.

Next, processing 300, which is a specific example of the processing in Step 212, is described in detail with reference to FIG. 3. The processing 300 may be implemented by the system 100 when the CPU 102 executes at least a part of the program instructions included in the CT image segmentation program 122.

Step 302 represents the start of the processing. In Step 304, data to be processed by the CT image segmentation program 122 is read (loaded). Specifically, all or a part of the CT images 130 is read from the mass storage device 106 and stored in the main storage device 104. In some embodiments, the CT image 130 may be directly copied to the main storage device 104 from an external device through the peripheral device interface 108 or the network interface 109.

In the CT image 130 to be processed by the processing 300 in the present example, pixel values representing water are corrected to be zero in advance. Thus, the CT image 130 has some pixels having negative pixel values.

Next, the processing 300 creates a histogram of pixel values in the CT image 130 (Step 308). In some embodiments, a histogram may be created after a bed portion is removed from the CT image 130. Step 306 represents a step for creating a reference image used to remove the bed portion. Step 306 is performed as follows.

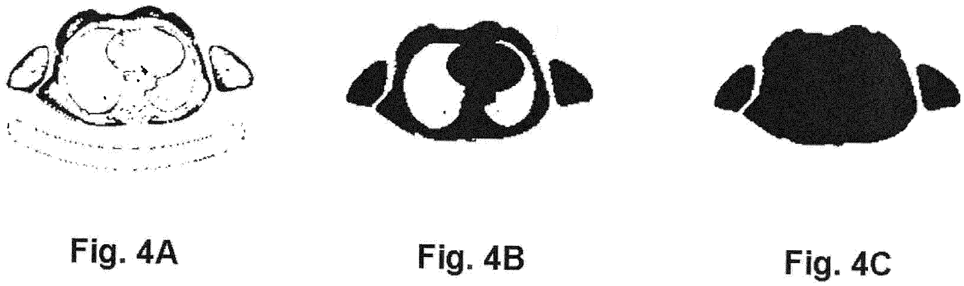

(Step 306A) First, the processing 300 creates a binary image obtained by binarizing the CT image 130. A threshold for the binarization is a threshold with which a human body is highly likely to be extracted. For example, in the present example, the CT image 130 is corrected in advance such that pixel values representing water are zero as described above, and hence, for example, a binary image may be created by using -190 as a threshold. FIG. 4A illustrates an example of the created binary image.

(Step 306B) Next, the processing 300 searches a vertical slice in the body axis direction from the center of the created binary image for a location with data. Then, region growing processing is performed from the location to create a binary image from which the bed portion has been removed. FIG. 4B illustrates a binary image created by processing the binary image in FIG. 4A in this manner.

When region growing has failed, a binary image is created by removing a bed by 3D labeling processing. The largest label is regarded as a human body, and portions other than the human body are removed to extract only a human body portion.

(Step 306C) After the bed is removed, filling processing to fill hole portions in the body with values is performed, and opening processing of morphology operation is performed to remove a bed that has not been completely removed. FIG. 4C illustrates an image created by processing the binary image in FIG. 4B in this manner. The image obtained by the processing in Step 306C is referred to as "reference image".

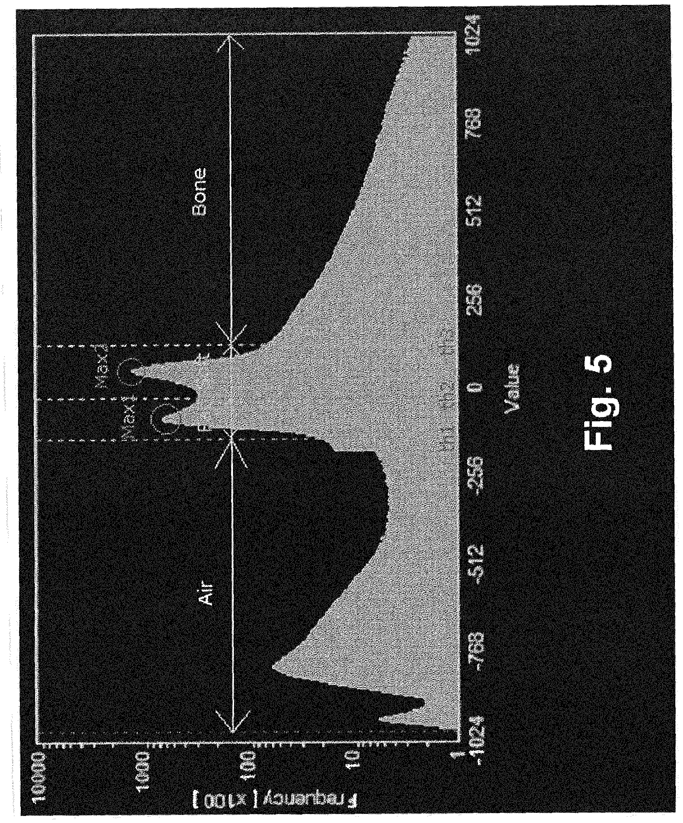

In Step 308, as described above, the processing 300 creates a histogram of pixel values in the CT image 130. In the present example, the histogram is created by using only pixels where data is present in corresponding pixels in the reference image created in Step 306 among pixels in the CT image 130. In an embodiment where Step 306 is not performed, a histogram may be created by using the entire CT image 130. FIG. 5 illustrates an example of the created histogram. As described above, the CT image 130 is corrected in advance such that pixel values representing water are zero. The CT image 130 is normalized in advance such that pixel values are distributed in the range of -1024 to +1024. Thus, the range of the horizontal axis (bin value) in the exemplified histogram is -1024 to +1024.

As exemplified in FIG. 5, in most cases, a histogram of a CT image has two peaks near a bin value corresponding to water (in the present example, a bin value 0). The peak of a bin value smaller than the bin value corresponding to water is a peak created by pixels corresponding to a fat region, and is herein referred to as "fat region peak". In FIG. 5, this peak is denoted by Max1. The peak of a bin value higher than the bin value corresponding to water is a peak created by pixels corresponding to soft tissue (such as muscles and brain), and is herein referred to as "soft region peak". In FIG. 5, this peak is denoted by Max2. When a subject carries little fat, the peak of the fat region may be unclear.

Bin values around the fat region peak are considered to correspond to the fat region. In FIG. 5, this region is indicated by Fat. Bin values around the soft region peak are considered to correspond to the soft tissue. In FIG. 5, this region is indicated by Soft. Bin values smaller than values corresponding to the fat region are considered to correspond to an air region. In FIG. 5, this region is indicated by Air. A region having a large number of pixels with CT values that are bin values of the air region is possibly a lung field. Bin values larger than values corresponding to the soft tissue region are considered to correspond to bones. In FIG. 5, this region is indicated by Bone. In FIG. 5, a boundary between the air region and the fat region, a boundary between the fat region and the soft tissue region, and a boundary between the soft tissue region and the bone region are denoted by th1, th2, and th3, respectively. In the subsequent steps, the fat region peak, the soft region peak, and th1, th2, and th3 are automatically determined.

In some embodiments, the histogram may be subjected to smoothing processing before the processing 300 proceeds to Step 310 from Step 308.

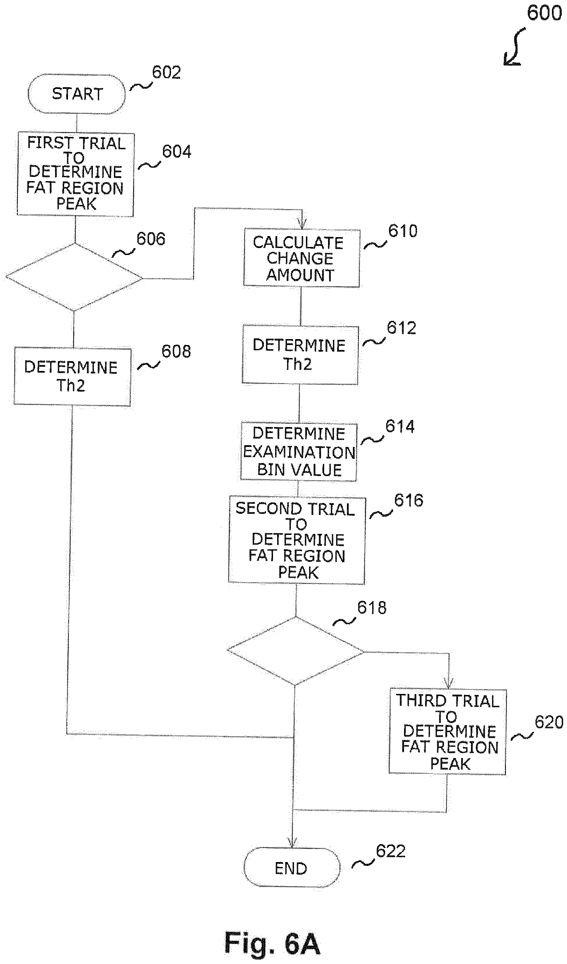

In Step 310, the processing 300 determines the above-mentioned fat region peak and a second threshold (th2 illustrated in FIG. 5), which is a threshold representing a boundary between the fat region and the soft tissue region. An example of the processing in Step 310 is described with reference to FIG. 6A.

FIG. 6A is a flowchart for describing processing 600, which is a specific example of the processing in Step 310. The processing 600 may be implemented by the system 100 when the CPU 102 executes at least a part of the program instructions included in the CT image segmentation program 122.

Step 602 represents the start of the processing. In Step 604, the processing 600 makes a first trial to determine a fat region peak in the histogram created in Step 308. Peak detection processing is not required to be performed in the entire range of bin values, but only needs to be performed in the range of bin values with which it has been experientially known that a fat region peak is present. For example, in a CT image corrected and normalized such that pixel values corresponding to water are 0 and the range of pixel values is -1024 to +1024, the inventors of the present invention have confirmed that the fat region peak of the histogram is distributed in the range of bin values of -256 to 0 in most cases. Thus, for example, the search range for a fat region peak may be set such that a lower limit bin value is, for example, -256 and an upper limit bin value is, for example, 0 (that is, a bin value corresponding to water), and a fat region peak may be searched for in the range.

In Step 604, the processing 600 searches for a maximum frequency value in the search range. When the maximum frequency value is not a frequency value of the bin value corresponding to water (in the present example, a bin value 0), the processing 600 determines that the maximum frequency value and the bin value corresponding to the maximum frequency value are respectively a frequency value and a bin value of the fat region peak.

However, when a subject, for which the CT image 130 has been taken, carries little fat, for example, the maximum frequency value in the search range may be a frequency value of a bin value 0 in some cases. In this case, the fat region peak is determined by using another method.

In Step 606, the processing 600 determines whether the fat region peak is successfully determined. When the fat region peak is successfully determined, the processing 600 proceeds to Step 608, and determines a second threshold that is a threshold representing a boundary between the fat region and the soft tissue region. In Step 608, the processing 600 determines the second threshold to be a bin value with which the frequency value is minimum in the range from the bin value of the fat region peak to a reference bin value (in the present example, a bin value 0). After that, the processing 600 proceeds to Step 622 to finish the processing.

When it is determined in Step 606 that the fat region peak has not been successfully determined, the processing 600 proceeds to Step 610. In Step 610, the processing 600 calculates a "change amount" defined by the following relational expressions for each bin value in the range from the lower limit bin value set in Step 604 to the reference bin value (in the present example, a bin value 0) that is the bin value corresponding to water:

$$\text{Difference of bin value } i \text{ from the previous bin value} = (\text{Frequency value of bin value } i{-}1) - (\text{Frequency value of bin value } i);$$

$$\text{Difference of bin value } i \text{ from the subsequent bin value} = (\text{Frequency value of bin value } i) - (\text{Frequency value of bin value } i{+}1);$$

and

$$\text{Change amount of bin value } i = (\text{Difference of bin value } i \text{ from the previous bin value})^2 + (\text{Difference of bin value } i \text{ from the subsequent bin value})^2$$

where i represents an integer.

Next, the processing 600 proceeds to Step 612. In this step, the processing 600 determines the above-mentioned second threshold that is a threshold representing the boundary between the fat region and the soft tissue region, to be a bin value with which the above-mentioned change amount is minimum in the range from the lower limit bin value to the reference bin value.

Subsequently, the processing 600 proceeds to Step 614. In this step, the processing 600 determines an examination bin value serving to be a lower limit bin value used to search for a fat region peak in the next step. The examination bin value is determined to be either of the following:

(a) a bin value with which the change amount calculated in Step 610 is maximum in the range from the lower limit bin value set in Step 604 to the second threshold set in Step 612; and
(b) a bin value corresponding to a frequency value that first falls below a frequency value of the second threshold among frequency values varying from the second threshold determined in Step 612 toward the lower limit bin value.

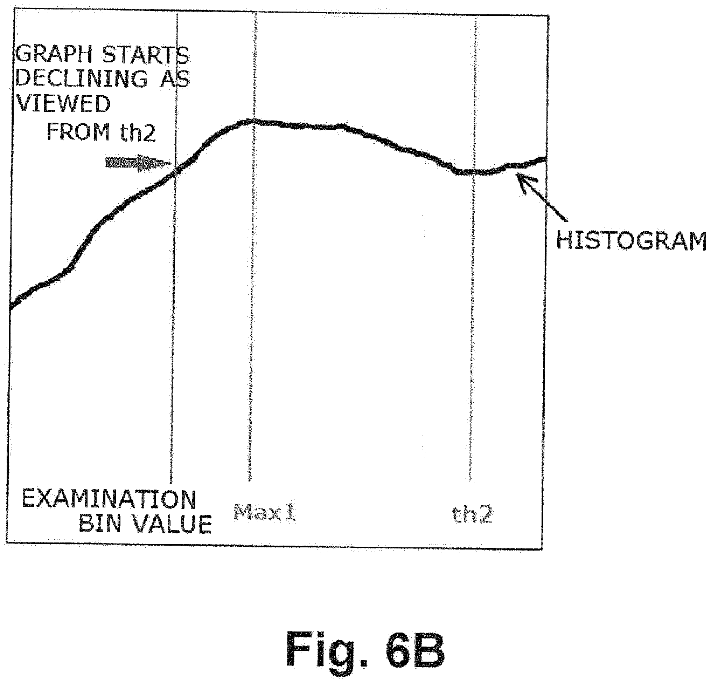

FIG. 6B illustrates how the examination bin value determined by (b) above appears.

In Step 616, the processing 600 makes a second trial to determine a fat region peak. In this step, the processing 600 determines that a bin value with which the value of "difference from the previous bin value" calculated in Step 610 is positive and minimum in the range from an examination boundary value to the second threshold and the frequency value corresponding to the bin value are respectively a bin value and a frequency value of the fat region peak.

In Step 618, the processing 600 determines whether the fat region peak is successfully determined. When the fat region peak is successfully determined, the processing 600 proceeds to Step 622 to finish the processing. When the fat region peak has not been successfully determined, such as when a bin value with which the value of the "difference from the previous bin value" is positive is not found in Step 616, the processing 600 proceeds to Step 620.

In Step 620, the processing 600 makes a third trial to determine a fat region peak. In this step, the processing 600 determines that a maximum frequency value in the range from the lower limit bin value set in Step 604 to the second threshold set in Step 612 and the bin value corresponding to the maximum frequency value are respectively a frequency value and a bin value of the fat region peak. The fat region peak and the second threshold can be determined without fail by the third trial. The processing 600 proceeds to Step 622 to finish the processing.

Subsequently, the description of the processing 300 is continued with reference back to FIG. 3. In Step 312, the processing 300 determines a soft region peak. Similarly to the fat region peak detection processing, soft region peak detection processing is not required to be performed in the entire range of bin values, but only needs to be performed in the range of bin values with which it has been experientially known that a soft region peak is present. For example, in a CT image corrected and normalized such that pixel values corresponding to water are 0 and the range of pixel values is -1024 to +1024, the inventors of the present invention have confirmed that a soft region peak of the histogram is distributed in the range of bin values of 0 to +256 in most cases. Thus, the soft region peak detection processing in Step 312 may be performed in the range of bin values from 0 to +256. Unlike the fat region, the soft tissue is considered to exist with a given frequency or more in any person, and hence, a soft region peak can be determined by this detection processing in most cases.

In Step 314, the processing 300 determines a first threshold (th1 illustrated in FIG. 5), which is a threshold representing a boundary between the air region and the fat region. Various methods can be used to determine the first threshold, and one example is the following method.

(314a) As the first threshold, the processing 300 employs a bin value that has a frequency value equal to or smaller than a predetermined proportion of the frequency value of the fat region peak or smaller than the predetermined proportion in the range from the lower limit bin value set in Step 604 to the second threshold set in Step 310. The inventors of the present invention have confirmed that 10%, for example, is one of the appropriate values for the predetermined proportion. However, the proportion may be another value such as 5%. The proportion may be determined for each CT device by using data on a plurality of subjects.

(314b) In some embodiments, the condition for determining the first threshold may include a condition that the absolute value of difference in frequency value from the previous bin value is equal to or smaller than a predetermined value. The inventors of the present invention have confirmed that 50, for example, is one of the appropriate values for the predetermined value. However, this value is merely illustrative, and various values can be employed in some embodiments.

(314c) In some embodiments, as the first threshold, the processing 300 may employ a bin value with which the same "change amount" as the one calculated in Step 610 is maximum in the range from the lower limit bin value set in Step 604 to the second threshold set in Step 310. In some embodiments, when there is no bin value satisfying the conditions 314a and 314b, the processing 300 may determine the first threshold by using the condition 314c.

In Step 316, the processing 300 determines a third threshold (th3 illustrated in FIG. 5), which is a threshold representing a boundary between the fat region and the bone region. Various methods can be used to determine the third threshold. In one example, the threshold may be defined as a bin value that is larger than the bin value corresponding to the soft region peak and has a frequency value accounting for a predetermined proportion of the frequency value of the soft region peak. The inventors of the present invention have confirmed that 5%, for example, is one of the appropriate values for the predetermined proportion. However, the proportion may be another value such as 10%. The proportion may be determined for each CT device by using data on a plurality of subjects.

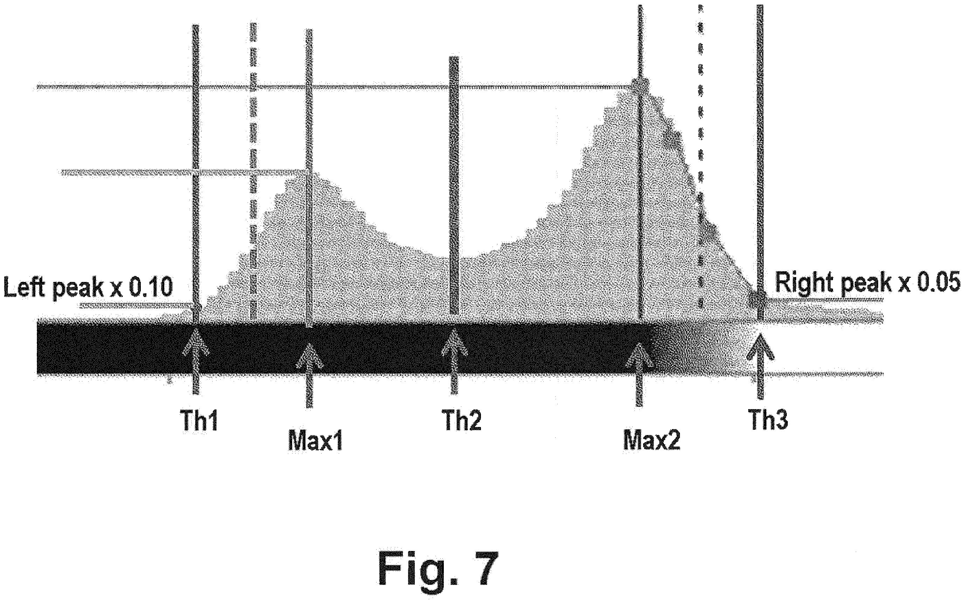

FIG. 7 schematically illustrates the relations among the histogram, the fat region peak, the soft region peak, and the first to third thresholds.

In Step 318, the processing 300 stores the first to third thresholds in a storage device. For example, the processing 300 may store the first to third thresholds in the mass storage device 106 as threshold data 140. Step 320 represents the end of the processing 300.

Next, processing 800, which is a specific example of the processing in Step 214, is described in detail with reference to FIG. 8. The processing 800 may be implemented by the system 100 when the CPU 102 executes at least a part of the program instructions included in the CT image segmentation program 122.

Step 802 represents the start of the processing. In Step 804, data to be processed by the CT image segmentation program 122 is loaded. Similarly to Step 304, the data to be loaded in this step is (all or a part of) the CT image 130. In addition, the threshold data 140 created and stored in Step 212 (Step 318) is also read in Step 804. In some embodiments, the CT image 130 and/or the threshold data 140 is already stored in the main storage device 102, and hence is not loaded again. In this case, the processing in Step 804 is unnecessary.

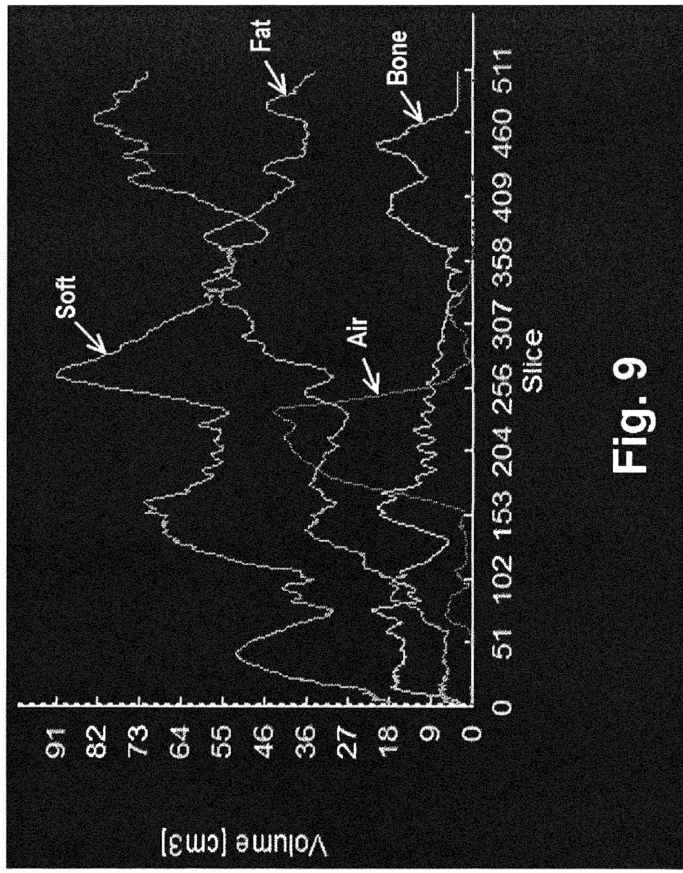

In Step 806, the processing 800 calculates, for axial section slices in the CT image, the volume of pixels belonging to the air region, the fat region, the soft tissue, and the bone region classified by the processing 300 to create graph data. FIG. 9 illustrates an example of the created graph displayed. The horizontal axis of the graph represents slice numbers of axial section slices in the CT image 130, and in the present example, a smaller number indicates the head side, and a larger number indicates the lower extremity side. The vertical axis of the graph represents volume ($cm^3$). The vertical axis may simply represent the number of pixels. A graph indicated by Air in FIG. 9 represents the volume of pixels (or the number of pixels) belonging to the air region. The pixels belonging to the air region are pixels with pixel values equal to or smaller than a first threshold or smaller than the first threshold. See the description of Step 314 for the first threshold. The graph is herein referred to as "air region volume graph".

A graph indicated by Fat in FIG. 9 represents the volume of pixels (or the number of pixels) belonging to the fat region. The pixels belonging to the fat region are pixels with pixel values included in the range from the first threshold to the second threshold. Whether the range includes a boundary (that is, the first threshold or the second threshold) depends on embodiments. See the description of Steps 314 and 310 for the first threshold and the second threshold. The graph is herein referred to as "fat region volume graph".

A graph indicated by Soft in FIG. 9 represents the volume of pixels (or the number of pixels) belonging to the soft tissue. The pixels belonging to the soft tissue are pixels with pixel values included in the range from the second threshold to the third threshold. Whether the range includes a boundary (that is, the second threshold or the third threshold) depends on embodiments. See the description of Steps 314 and 316 for the second threshold and the third threshold. The graph is herein referred to as "soft region volume graph".

A graph indicated by Bone in FIG. 9 represents the volume of pixels (or the number of pixels) belonging to the bone region. The pixels belonging to the bone region are pixels with pixel values equal to or larger than the third threshold or larger than the third threshold. See the description of Step 316 for the third threshold. The graph is herein referred to as "bone region volume graph".

Of the four graphs illustrated in FIG. 9, only the air region volume graph and the soft region volume graph are used in the processing 800. Thus, in some examples, only the two volume graphs may be created in Step 806. It should be noted that in the example of the processing 800, it is not necessarily required to display the graphs as illustrated in FIG. 9.

In some embodiments, the subsequent steps may be implemented after the graphs illustrated in FIG. 9 are subjected to smoothing processing.

In Step 808, the processing 800 determines a breast start slice that is an axial section slice located at an upper end of the breast in the CT image 130. In this step, the breast start slice is determined to be an axial section slice that is closer to the vertex than an axial section slice with the air region volume graph exhibiting a maximum value and has a volume value accounting for a predetermined proportion of the maximum value of the air region volume graph. The inventors of the present invention have confirmed that 10%, for example, is one of the appropriate values for the predetermined proportion, but the predetermined proportion is not limited to this value and can be changed in some embodiments.

In some examples, another condition that the difference in frequency from an adjacent slice is a predetermined proportion of the maximum value of the air region volume graph may be added to the determination condition for the breast start slice. The inventors of the present invention have confirmed that 1%, for example, is one of the appropriate values for the predetermined proportion, but the predetermined proportion is not limited to this value and can be changed in some embodiments.

In Step 810, the processing 800 determines an upper abdomen start slice located at an upper end of the upper abdomen in the CT image 130. In this step, the upper abdomen start slice is determined to be an axial section slice that is closer to the lower extremity than an axial section slice with the air region volume graph exhibiting a maximum value and with which the volume value of the soft region volume graph is the largest.

In Step 812, the processing 800 determines a neck start slice located at an upper end of the neck in the CT image 130. In this step, the neck start slice is determined to be an axial section slice that is located closer to the vertex than the breast start slice and with which the volume value of the air region volume graph is the largest.

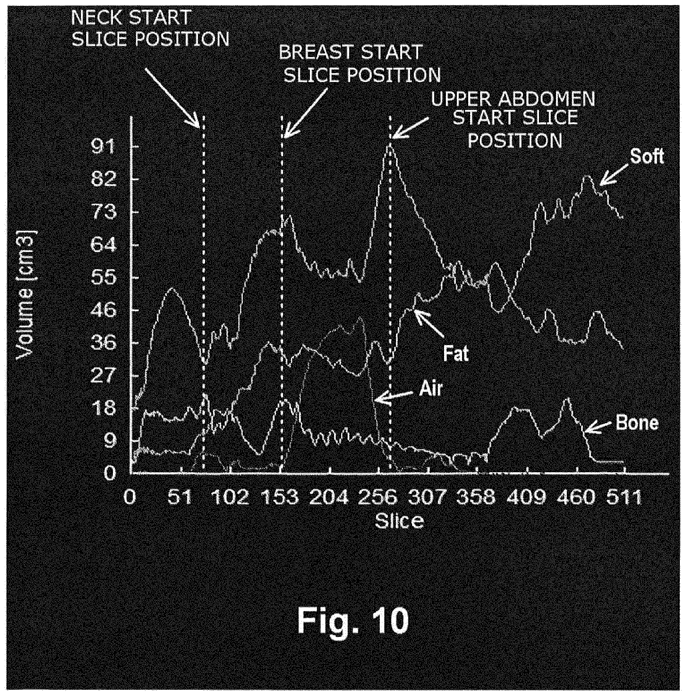

FIG. 10 illustrates the positions of the breast start slice determined in Step 808, the upper abdomen start slice determined in Step 810, and the neck start slice determined in Step 812 on the graphs in FIG. 9.

In Step 814, the processing 800 determines a head start slice located at an upper end of the head in the CT image 130. The determination processing is performed as follows:

(814a) performing 3D labeling on axial section slices on the vertex side of the neck start slice;

(814b) determining a head label that is a label located at the center in the axial section slices; and

(814c) calculating, for axial section slices from the neck start slice to the vertex side, the volume of a pixel group that is located in a region corresponding to the head label and has pixel values between the second threshold and the third threshold, and determining an axial section slice having the volume that first becomes zero to be a head start slice.

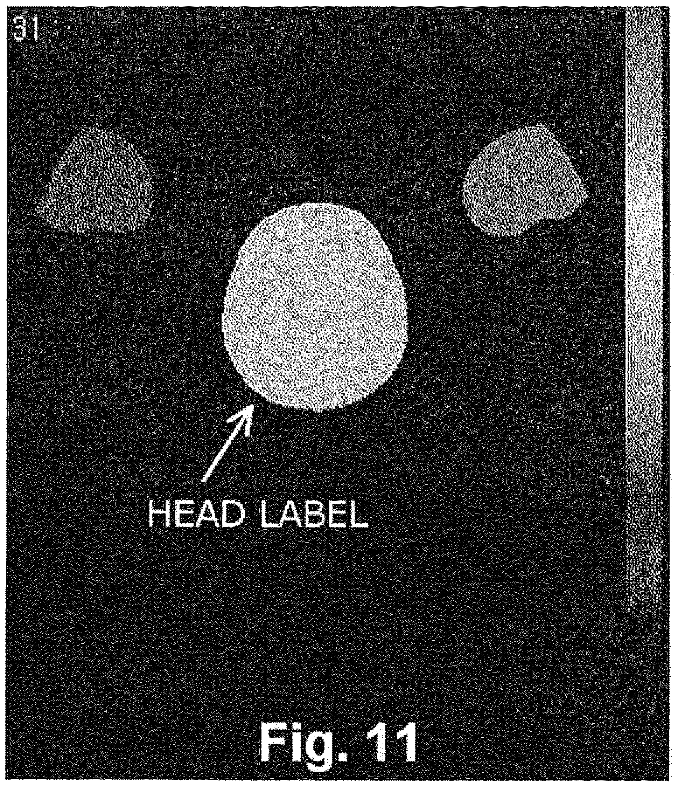

FIG. 11 illustrates an example of a head label located at a given axial section slice.

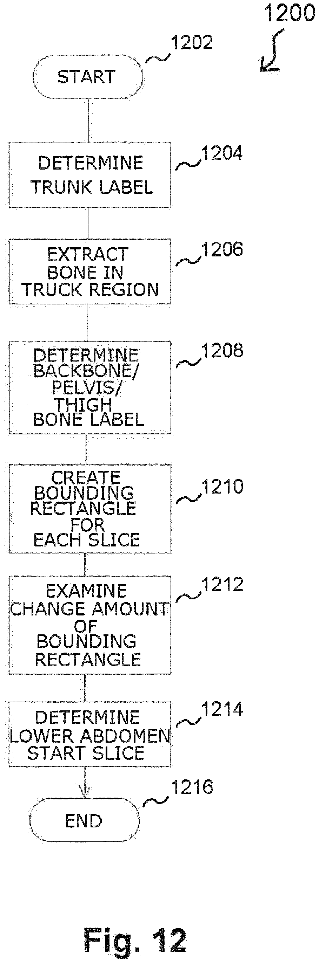

Subsequently, in Step 816, the processing 800 determines a lower abdomen start slice located at an upper end of the lower abdomen in the CT image 130. An example of the determination processing is described with reference to FIGS. 12 to 14.

Processing 1200 in FIG. 12 is a specific example of Step 816 in FIG. 8. The processing 1200 may also be implemented by the system 100 when the CPU 102 executes at least a part of the program instructions included in the CT image segmentation program 122.

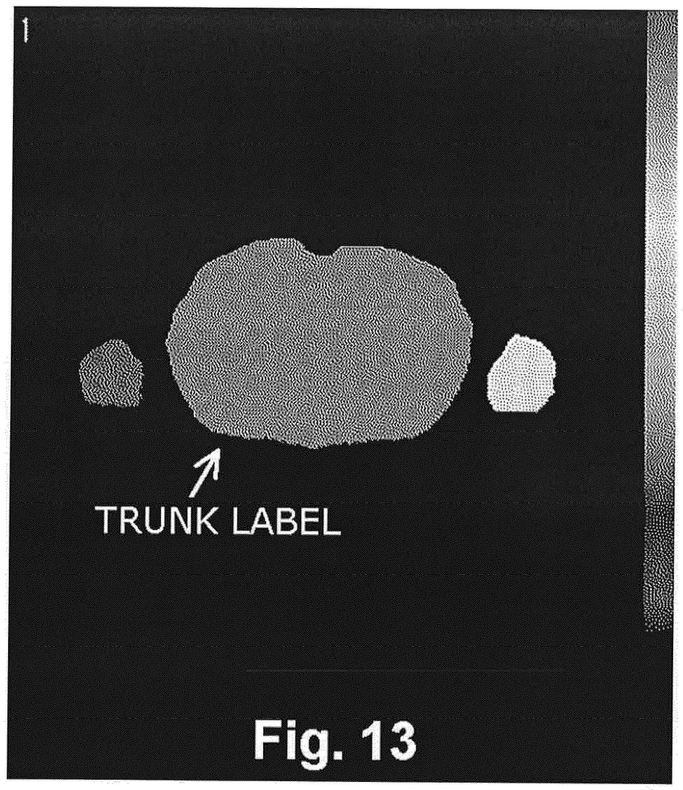

Step 1202 represents the start of the processing. In Step 1204, the processing 1200 performs 3D labeling on axial section slices on the lower extremity side of the upper abdomen start slice determined in Step 810 among axial section slices in the CT image 130, and determines a trunk label, which is the largest label. FIG. 13 illustrates how the determined trunk label appears by taking a given axial section slice as an example.

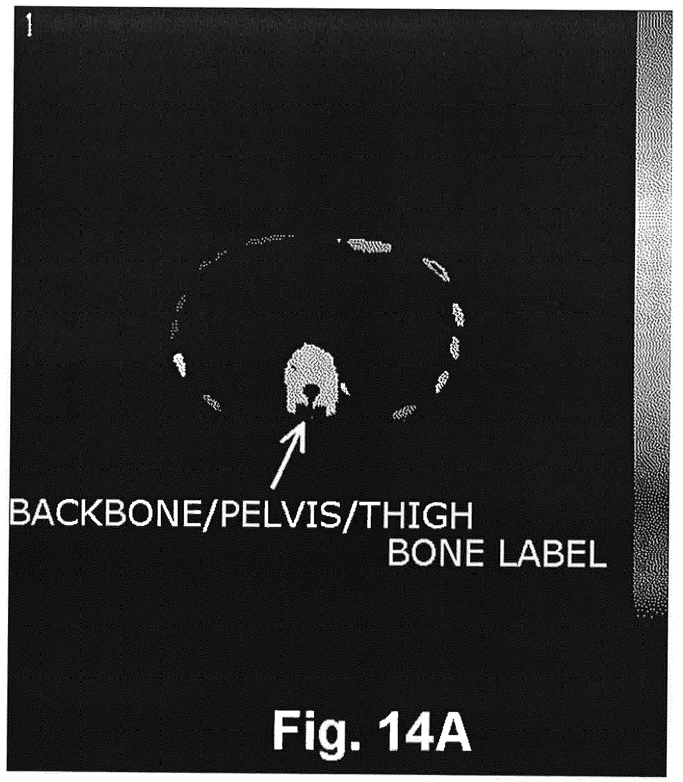

In Step 1206, the processing 1200 extracts, for the axial section slices on the lower extremity side of the upper abdomen start slice, a pixel group that is located in a region corresponding to the trunk label and has pixel values equal to or larger than the third threshold or larger than the third threshold. See the description of Step 316 for the third threshold. The extracted pixel group represents a bone region on the lower extremity side of the upper abdomen start slice. Subsequently, in Step 1208, the processing 1200 performs 3D labeling on the extracted pixel group to determine a backbone/pelvis/thigh bone label, which is the largest label. FIG. 14A illustrates how the determined backbone/pelvis/thigh bone label appears by taking a given axial section slice as an example.

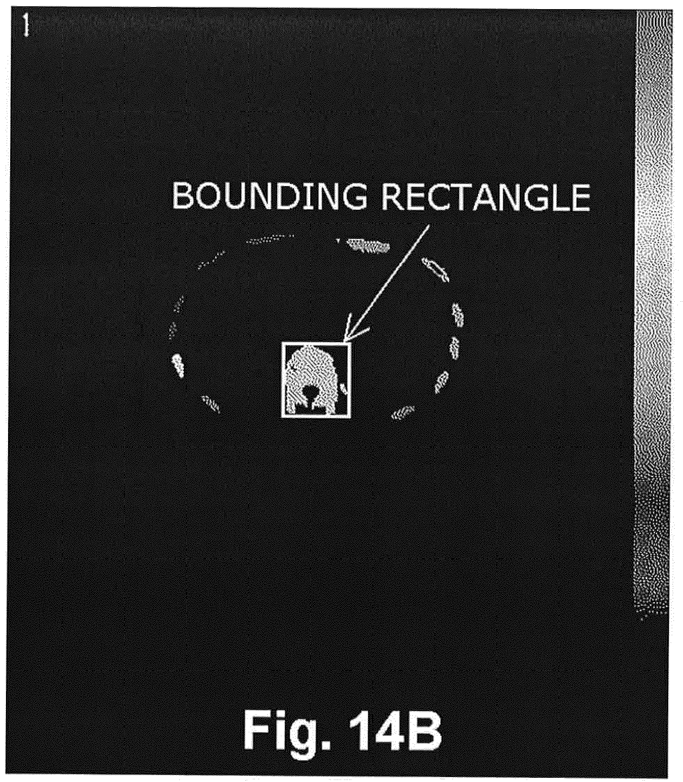

In Step 1210, the processing 1200 creates a bounding rectangle of a pixel group that corresponds to the backbone/pelvis/thigh bone label and has pixel values equal to or larger than the third threshold or larger than the third threshold for axial section slices on the lower extremity side of the upper abdomen start slice. FIG. 14B illustrates an example in which the bounding rectangle is superimposed and displayed on the backbone/pelvis/thigh bone label in FIG. 14A. In Step 1212, the processing 1200 examines a change amount of the area of the created bounding rectangle. In Step 1214, the processing 1200 determines an axial section slice having the largest change amount to be a lower abdomen start slice.

In some embodiments, the processing 1200 may examine a change amount of a bone irrespective of the bounding rectangle and determine a lower abdomen start slice in accordance with the result. For example, the processing 1200 may calculate the size (number of pixels) of a backbone/pelvis/thigh bone label for each slice, and determine a slice having the largest difference in size from an adjacent slice to be a lower abdomen start slice. Alternatively, irrespective of the change amount, the processing 1200 may determine a lower abdomen start slice depending on a threshold. For example, the processing 1200 may determine a lower abdomen start slice depending on whether the above-mentioned area or the size of the label falls below a predetermined threshold. The predetermined threshold may be determined from data on a plurality of subjects (for example, the average value thereof), or may be determined based on the maximum value of the above-mentioned area or label. For example, a slice in which the above-mentioned area or label accounts for a predetermined proportion of the maximum value may be determined to be a lower abdomen start slice.

Step 1216 represents the end of the processing.

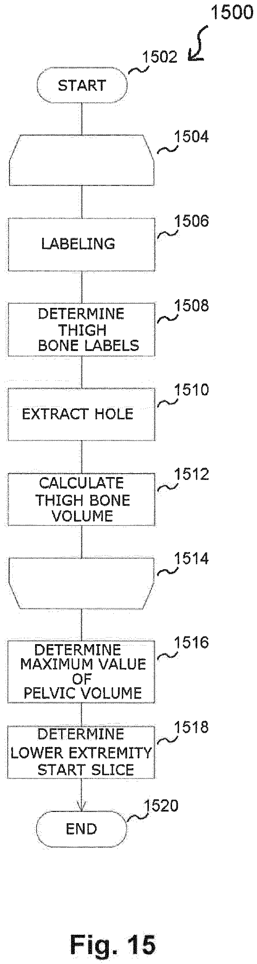

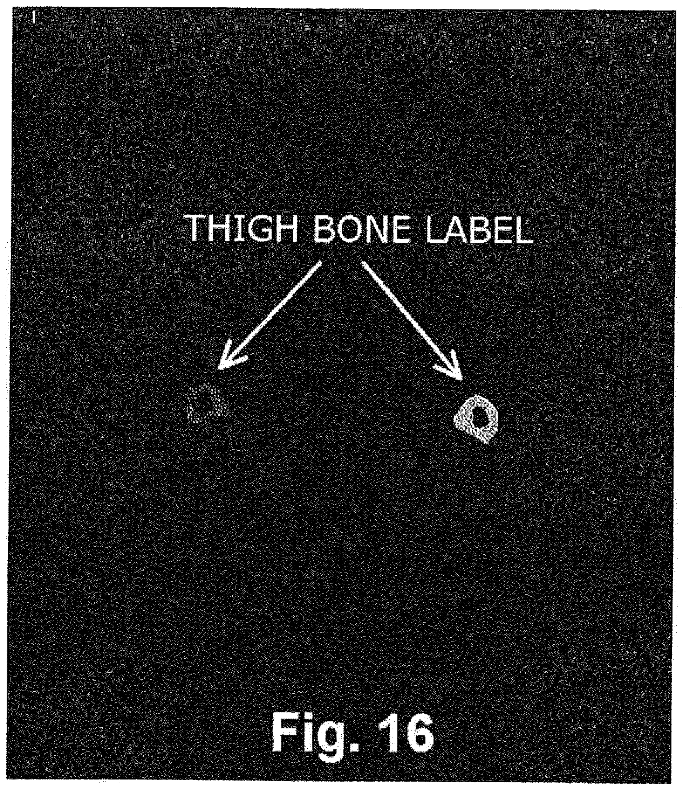

Referring back to FIG. 8, in Step 818, the processing 800 determines a lower extremity start slice located at an upper end of the lower extremity in the CT image 130. An example of the determination processing is described with reference to FIGS. 15 and 16.

Processing 1500 in FIG. 15 is a specific example of Step 818 in FIG. 8. The processing 1500 may be implemented by the system 100 when the CPU 102 executes at least a part of the program instructions included in the CT image segmentation program 122.

Step 1502 represents the start of the processing. In the loop denoted by reference numerals 1504 to 1514, the processing 1500 performs processing denoted by reference numerals 1506 to 1512 for axial section slices on the lower extremity side from the lower abdomen start slice determined in Step 816 among axial section slices in the CT image 130.

In Step 1506, the processing 1500 labels, for the current axial section slice in the loop, a pixel group that is located in a region corresponding to the trunk label determined in Step 1204 and has pixel values equal to or larger than the third threshold or larger than the third threshold. See the description of Step 316 for the third threshold. The processing 1500 extracts two labels having the largest and second largest pixel values, and when the two labels do not overlap with each other, determines the two labels to be thigh bone labels (Step 1508). FIG. 16 illustrates how the thigh bone labels appear by taking a given axial section slice as an example.

When the thigh bone labels are successfully determined, the processing 1500 extracts a hole for each of the thigh bone labels (Step 1510). When a hole is successfully extracted, the processing 1500 calculates, for the current axial section slice in the loop, a thigh bone volume that is the volume (volume or number of pixels) of a pixel group that is located in a region corresponding to the thigh bone labels and has pixel values equal to or larger than the third threshold or larger than the third threshold (Step 1512).

After exiting from the loop denoted by reference numerals 1504 to 1514, the processing 1500 proceeds to Step 1516. In Step 1516, the processing 1500 determines a maximum value of a pelvic volume in axial section slices on the lower extremity side of the lower abdomen start slice. The determination may be made by calculating, for axial section slices on the lower extremity side of the lower abdomen start slice, the volume (volume or number of pixels) of a pixel group that is located in a region corresponding to the trunk label determined in Step 1204 and has pixel values equal to or larger than the third threshold or larger than the third threshold, and specifying a maximum value of the calculated volume.

In Step 1518, the processing 1500 determines a lower extremity start slice. The lower extremity start slice is determined to be a slice having the value of the thigh bone volume accounting for a predetermined proportion of the pelvic volume maximum value determined in Step 1516 first out of the axial section slices for which the thigh bone volume is successfully calculated among axial section slices on the lower extremity side of the lower abdomen start slice. The inventors of the present invention have confirmed that, for example, a half or less than a half of the pelvic volume maximum value is one of the appropriate values for the predetermined proportion. However, in some embodiments, another value may be employed. Step 1520 represents the end of the processing.

Referring back to FIG. 8, in Step 820, slice position information on the breast start slice, the upper abdomen start slice, the neck start slice, the head start slice, the lower abdomen start slice, and the lower extremity start slice determined in Steps 808 to 818 is stored in storage means in the system 100. For example, as illustrated in FIG. 1, the slice position information may be stored in the mass storage device 106 as the slice position information 142. In some embodiments, the slice position information may be stored in the main storage device 104. For example, the slice position information may be slice numbers of axial section slices in the CT image 130. Step 822 represents the end of the processing 800.

Subsequently, a specific example of the processing in Step 216 in FIG. 2 is described with reference to FIG. 17.

FIG. 17 is a diagram for describing processing 1700, which is a specific example of the processing in Step 216 in FIG. 2. The processing 1700 may be implemented by the system 100 when the CPU 102 executes at least a part of the program instructions included in the physiological accumulation removal program 124.

Step 1702 represents the start of the processing. In Step 1704, data to be processed by the physiological accumulation removal program 124 is read (loaded). The data to be loaded in this step is (whole or a part of) a nuclear medical image (in the present example, a PET image) 132. Additionally, in Step 1708, the slice position information 142 created and stored by the processing 800 is also read. In some embodiments, the nuclear medical image 132 and/or the slice position information 142 is already stored in the main storage device 102 and is thus not loaded again. In this case, the processing in Step 1704 is unnecessary.

As described above, the slice position information 142 includes slice position information (slice number) on at least one of the breast start slice, the upper abdomen start slice, the neck start slice, the head start slice, the lower abdomen start slice, and the lower extremity start slice. The slice position information is created based on a CT image 130 registered with the nuclear medical image 132. Thus, the use of the slice position information enables the breast start slice, the upper abdomen start slice, and the like to be specified also in the nuclear medical image 132. Specifically, for example, in the nuclear medical image 132, an axial section slice having the same slice number as the breast start slice number included in the slice position information 142 can be regarded as a slice located at an upper end of the breast in the nuclear medical image 132. In other words, an axial section slice having the same slice number as the breast start slice number included in the slice position information 142 can be regarded as the breast start slice also in the nuclear medical image 132. The same holds true for the upper abdomen start slice, the neck start slice, the head start slice, the lower abdomen start slice, and the lower extremity start slice. In the following description of the processing 1700, it should be understood that the terms of the breast start slice, the upper abdomen start slice, the neck start slice, the head start slice, the lower abdomen start slice, and the lower extremity start slice all represent axial section slices in the nuclear medical image 132.

In Steps 1706 to 1710, the processing 1700 performs processing for determining a high accumulation part of the head in the nuclear medical image 132 registered with the CT image 130. In Step 1706, the processing 1700 uses the slice position of a head start slice and the slice position of a neck start slice to set a search region for a pixel having the maximum pixel value in the nuclear medical image 132. In one example, in the nuclear medical image 13, pixels included in all axial section slices from the head start slice to the neck start slice may be set as a search region. In some embodiments, however, the search region may be narrowed in order to improve processing speed and memory consumption. The inventors of the present invention have confirmed that appropriate results are obtained with a search region defined as follows:

- the axial direction ranges from an intermediate slice between the head start slice and the neck start slice to the head start slice;

- the sagittal direction covers the entire range, that is, from 0 to the matrix size in the sagittal section slice; and

- the coronal direction covers only the center in the horizontal direction.

The definition of the search region may vary in other embodiments.

In Step 1708, the processing 1700 determines a pixel having the maximum pixel value in the nuclear medical image 132 in the search region set in Step 1706.

In Step 1710, the processing 1700 performs region growing from the pixel having the maximum pixel value determined in Step 1708. A region obtained by region growing is determined to be a high accumulation region in the head.

In Steps 1712 to 1716, the processing 1700 performs processing for determining a high accumulation part of the bladder in the nuclear medical image 132 registered with the CT image 130. In Step 1712, the processing 1700 uses the slice position of a lower abdomen start slice and the slice position of a lower extremity start slice to set a search region for a pixel having the maximum pixel value in the nuclear medical image 132. In one example, in the nuclear medical image 132, pixels included in all axial section slices between the lower abdomen start slice and the lower extremity start slice may be set as a search region. In some embodiments, however, the search region may be narrowed in order to improve processing speed and memory consumption. The inventors of the present invention have confirmed that appropriate results are obtained with a search region defined as follows:

- the axial direction ranges from an intermediate slice between the lower abdomen start slice and the lower extremity start slice to the lower extremity start slice;

- the sagittal direction covers the entire range, that is, from 0 to the matrix size in the sagittal section slice; and

- the coronal direction covers only the center in the horizontal direction.

The definition of the search region may vary in other embodiments.

In Step 1714, the processing 1700 determines a pixel having the maximum pixel value in the nuclear medical image 132 in the search region set in Step 1712.

In Step 1716, the processing 1700 performs region growing from the pixel having the maximum pixel value determined in Step 1714. A region obtained by region growing is determined to be a high accumulation part of the bladder.

In Step 1718, data on the high accumulation region in the head obtained in Step 1710 and data on the high accumulation region in the bladder obtained in Step 1716 are stored in storage means in the system 100. For example, the data may be stored in the mass storage device 106 as region data 144.

Referring back to FIG. 2, in Step 220, the high accumulation region in the head and the high accumulation region in the bladder are masked based on the result in Step 216, and the nuclear medical image 132 is displayed. FIG. 18 illustrates a display example.

FIG. 18 illustrates one screen in an implementation example of the system 100. On the screen, a display 1802 of a given coronal section in the CT image 130 and a display 1804 of a corresponding coronal section in the nuclear medical image 132 appear. As illustrated in FIG. 18, the boundary of the body region is indicated in the image 1802 based on the slice position information 142. When an operator operates a button 1808 of a user interface with a mouse or the like, an image 1806 appears in which a high accumulation region 1810 in the head and a high accumulation region 1812 in the bladder in the image 1804 are masked based on the region data 144.

Because the high accumulation region 1810 in the head and the high accumulation region 1812 in the bladder are masked, the nuclear medical image can be observed without being interfered by unnecessary signals from the head and the bladder.

While the invention in the present application has been described above in detail by way of preferred examples, the above description and the accompanying drawings are not presented for the purpose of limiting the scope of the invention in the present application, but are presented in order to meet the legal requirements. The embodiments of the invention in the present application have various variations other than the ones introduced herein. For example, various kinds of numerical values indicated in the specification or the drawings are all illustrative, and these numerical values are not presented for the purpose of limiting the scope of the invention. Individual features included in various kinds of examples introduced in the specification or the drawings are not limited to usage with examples in which these features are explicitly described to be included, but may be used in combination with other examples described herein or various kinds of specific examples that are not described herein. In particular, the processing illustrated in each of the flowcharts is not necessarily required to be executed in the order stated herein. According to the preference of an implementor or if necessary, the processing may be executed in another order or simultaneously in parallel, and a plurality of blocks may be implemented integrally or in a loop as appropriate. These variations are all included in the scope of the invention disclosed herein, and the scope of the invention is not limited by the embodiments of the processing. The described order of the processing defined in the claims is not necessarily required to specify the essential order of the processing. For example, an embodiment specifying a different order of the processing and an embodiment that executes the processing in a loop are also included in the scope of the invention as specified in the claims.

In addition, for example, embodiments of the CT image segmentation program 122 and the physiological accumulation removal program 124 include an embodiment in which these programs are each a single computer program and an embodiment in which these programs are a program group including a plurality of independent computer programs. In some embodiments, the two computer programs are provided as a single computer program. In some embodiments, the computer programs are provided with the registration program 120 incorporated. Various embodiments of computer programs are applicable as well known, and these variations are all included in the scope of the invention disclosed herein. It should be noted that the applicant claims the right to have a patent granted on all the embodiments not deviating from the spirit of the invention disclosed herein regardless of whether a patent is claimed in the current set of the appended claims.

**Reference Signs List**

100 system
102 CPU
104 main storage device
106 mass storage device
107 display interface
108 peripheral device interface
109 network interface
120 registration program
122 CT image segmentation program
124 physiological accumulation removal program
130 CT image data
132 nuclear medical image data

**Claims**

**1.** A method for automatically classifying pixel values in a computed tomography (CT) image, the method comprising:

creating a histogram of pixel values based on the CT image;
determining a fat region peak that is a peak in a fat region in the histogram;
determining a soft region peak that is a peak in a soft tissue region in the histogram;
determining a first threshold that is a threshold representing an upper limit of bin values in an air region, based on a frequency value of the fat region peak in a region of bin values smaller than a bin value of the fat region peak;
determining a second threshold that is a threshold representing a bin value of a boundary between the fat region and the soft tissue region; and
determining a third threshold that is a threshold representing a lower limit of bin values in a bone region, based on a frequency value of the soft region peak in a region of bin values larger than a bin value corresponding to the soft region peak.

2. The method according to claim 1, wherein the creating of a histogram comprises:

   creating a reference image that is a binary image from which a bed portion is removed to leave only a human body portion in the CT image; and
   creating the histogram by using only pixels where data is present in corresponding pixels in the reference image among pixels in the CT image.

3. The method according to claim 1 or 2, comprising performing smoothing processing on the histogram before the determining of a fat region peak and the determining of a soft region peak.

4. The method according to any one of claims 1 to 3, wherein
   the determining of a fat region peak comprises determining a maximum frequency value in a range from a lower limit bin value that is set so as to include the fat region peak to a reference bin value that is a bin value corresponding to water, and when the maximum frequency value is not a frequency value of the reference bin value, determining that the maximum frequency value and the bin value corresponding to the maximum frequency value are respectively a frequency value and a bin value of the fat region peak, and
   the method further comprises determining the second threshold to be a bin value with which a frequency value is minimum in a range from the bin value of the fat region peak to the reference bin value.

5. The method according to claim 4, wherein when the maximum frequency value in the range from the lower limit bin value to the reference bin value is a frequency value of the reference bin value, the determining of a fat region peak comprises:

   determining the second threshold to be a bin value with which a change amount of a frequency value is minimum in the range from the lower limit bin value to the reference bin value, where the change amount being a value defined as:

   Difference of bin value i from previous bin value = (Frequency value of bin value i-1) - (Frequency value of bin value i);

   Difference of bin value i from subsequent bin value = (Frequency value of bin value i) - (Frequency value of bin value i+1);

   and

   Change amount of bin value i = (Difference of bin value i from previous bin value)$^2$ + (Difference of bin value i from subsequent bin value)$^2$

   where i is an integer;
   determining an examination bin value to be either of:

   - a bin value having the maximum change amount in the range from the lower limit bin value to the second threshold; and
   - a bin value corresponding to a frequency value that first falls below a frequency value of the second threshold among frequency values varying from the second threshold toward the lower limit bin value; and

   determining that a bin value with which a value of the difference from the previous bin value is positive and minimum in the range from an examination boundary value to the second threshold and the frequency value corresponding to the bin value are respectively a bin value and a frequency value of the fat region peak.

6. The method according to claim 5, wherein when a bin value with which the value of the difference from the previous bin value is not found in the range from the examination boundary value to the second threshold, the determining of a fat region peak comprises determining that a maximum frequency value in the range from the lower limit bin value to the second threshold and the bin value corresponding to the maximum frequency value are respectively a frequency value and a bin value of the fat region peak.

7. The method according to claim 5 or 6, wherein the determining of a soft region peak comprises performing peak detection processing in a predetermined bin value range.

8. The method according to claim 7, wherein
the first threshold is determined to be either of:

- a bin value that is smaller than a bin value of the fat region peak and has a frequency value equal to or smaller than a predetermined proportion of a frequency value of the fat region peak or smaller than the predetermined proportion in the range from the lower limit bin value to the second threshold; and
- a bin value having the maximum change amount in the range from the lower limit bin value to the bin value of the fat region peak; and

the third threshold is determined to be a bin value that is larger than a bin value corresponding to the soft region peak and has a frequency value accounting for a predetermined proportion of the frequency value of the soft region peak.

9. A method for automatically segmenting a region of the CT image for which the first to third thresholds are determined by the method as claimed in any one of claims 1 to 8, the method comprising:

creating an air region volume graph that is a graph having one axis representing slice numbers of axial section slices in the CT image and another axis representing the volume of at least a part of a pixel group with pixel values equal to or smaller than the first threshold or smaller than the first threshold in slices corresponding to the slice numbers; and
determining an axial section slice that is closer to a vertex than an axial section slice for which the air region volume graph exhibits a maximum value, the axial section slice having a volume value accounting for a predetermined proportion of the maximum value of the air region volume graph, to be a breast start slice located at an upper end of a breast.

10. The method according to claim 9, wherein the air region volume graph is subjected to smoothing processing before the breast start slice is determined.

11. The method according to claim 9 or 10, further comprising:

creating a soft region volume graph that is a graph having one axis representing slice numbers of axial section slices in the CT image and another axis representing the volume of a pixel group with pixel values between the second threshold and the third threshold in slices corresponding to the slice numbers; and
determining an axial section slice that is closer to a lower extremity than an axial section slice for which the air region volume graph exhibits a maximum value, the axial section slice having the largest volume value of the soft region volume graph, to be an upper abdomen start slice located at an upper end of an upper abdomen.

12. The method according to claim 11, wherein the soft region volume graph is subjected to smoothing processing before the upper abdomen start slice is determined.

13. The method according to any one of claims 9 to 12, further comprising determining an axial section slice that is closer to a vertex than the breast start slice and has the largest volume value of the air region volume graph to be a neck start slice located at an upper end of a neck.

14. The method according to claim 13, further comprising:

performing 3D labeling on axial section slices closer to the vertex than the neck start slice;
determining a head label that is a label located at a center in the axial section slice; and
calculating, for axial section slices from the neck start slice to the vertex side, the volume of a pixel group that is located in a region corresponding to the head label and has pixel values between the second threshold and the third threshold, and determining an axial section slice having the volume that first becomes zero to be a head start slice located at an upper end of a head.

15. The method according to any one of claims 11 and 12 or claims 13 and 14 depending from claim 11 or 12, further comprising determining, for axial section slices closer to the lower extremity than the upper abdomen start slice, a lower abdomen start slice located at an upper end of a lower abdomen based on a change in the volume of a bone.

16. The method according to claim 15, wherein the determining of a lower abdomen start slice comprises:

performing 3D labeling on axial section slices closer to the lower extremity than the upper abdomen start slice, and determining a trunk label, which is the largest label;
extracting, for the axial section slices close to the lower extremity than the upper abdomen start slice, a pixel group that is located in a region corresponding to the trunk label and has pixel values equal to or larger than the third threshold or larger than the third threshold, and performing 3D labeling on the extracted pixel group to determine a backbone/pelvis/thigh bone label, which is the largest label;
creating, for axial section slices closer to the lower extremity than the upper abdomen start slice, a bounding rectangle of a pixel group that corresponds to the backbone/pelvis/thigh bone label and has pixel values equal to or larger than the third threshold or larger than the third threshold; and
determining an axial section slice having the largest change amount of the bounding rectangle to be the lower abdomen start slice located at the upper end of the lower abdomen.

17. The method according to claim 16, further comprising:

performing, for axial section slices closer to the lower extremity than the lower abdomen start slice, the following:

labeling a pixel group that is located in a region corresponding to the trunk label and has pixel values equal to or larger than the third threshold or larger than the third threshold;
extracting two labels having largest and second largest sizes in the same slice, and when the two labels do not overlap with each other, determining the two labels to be thigh bone labels;
extracting, when the thigh bone labels are successfully determined, a hole for each of the thigh bone labels; and
calculating, when the hole is successfully extracted, a thigh bone volume that is the volume of a pixel group that is located in a region corresponding to the thigh bone label and has pixel values equal to or larger than the third threshold or larger than the third threshold;

calculating a maximum value of the volume of a pixel group that is located in a region corresponding to the trunk label and has pixel values equal to or larger than the third threshold or larger than the third threshold in the axial section slices closer to the lower extremity than the lower abdomen start slice; and
determining a slice having a value of the thigh bone volume accounting for a predetermined proportion of the maximum value first out of the axial section slices for which the thigh bone volume is successfully calculated among the axial section slices closer to the lower extremity than the lower abdomen start slice to be a lower extremity start slice located at an upper end of the lower extremity.

18. The method according to any one of claims 1 to 17, wherein the method is performed on a CT image registered with a nuclear medical image.

19. A method for automatically removing physiological accumulation from a nuclear medical image, the method comprising:

reading information on a slice position of the head start slice and information on a slice position of the neck start slice, the slices being determined by the method as claimed in claim 17 depending from claim 14;
setting a maximum pixel value search region in the nuclear medical image by using the slice position of the head start slice and the slice position of the neck start slice;
determining a pixel having a maximum pixel value in the maximum pixel value search region; and
determining a high accumulation region in the head by an approach of region growing from the pixel having the maximum pixel value.

20. The method according to claim 19, further comprising displaying the nuclear medical image while masking the determined brain region.

# EP 3 443 907 A1

21. A method for automatically removing physiological accumulation from a nuclear medical image, the method comprising:

reading information on a slice position of the lower abdomen start slice and information on a slice position of the lower extremity start slice, the slices being determined by the method as claimed in any one of claims 17 to 19 depending from claim 16;
setting a maximum pixel value search region in the nuclear medical image by using the slice position of the lower abdomen start slice and the slice position of the lower extremity start slice;
determining a pixel having a maximum pixel value in the maximum pixel value search region; and
determining a high accumulation region in a bladder by an approach of region growing from the pixel having the maximum pixel value.

22. The method according to claim 21, further comprising displaying the nuclear medical image while masking the determined bladder region.

23. A device comprising:

processing means; and
storage means,
the storage means including a program instruction,
the program instruction being configured to cause the device to implement the method according to any one of claims 1 to 22 when executed by the processing means.

24. A computer program comprising a program instruction configured to cause a device to implement the method according to any one of claims 1 to 22 when executed by means in the device.

**Fig. 1**

204

200

START

208

REGISTRATION

212

CT IMAGE
HISTOGRAM
SEGMENTATION

214

CT IMAGE
SEGMENTATION

216

SPECIFY
PET IMAGE
PHYSIOLOGICAL
ACCUMULATION

220

MASK
PROCESSING
AND
DISPLAY

224

END

# Fig. 2

Fig. 3

Fig. 4C

Fig. 4B

Fig. 4A

**Fig. 5**

EP 3 443 907 A1

600

START 602

FIRST TRIAL TO DETERMINE FAT REGION PEAK 604

606

DETERMINE Th2 608

CALCULATE CHANGE AMOUNT 610

DETERMINE Th2 612

DETERMINE EXAMINATION BIN VALUE 614

SECOND TRIAL TO DETERMINE FAT REGION PEAK 616

618

THIRD TRIAL TO DETERMINE FAT REGION PEAK 620

END 622

Fig. 6A

HISTOGRAM

GRAPH STARTS DECLINING AS VIEWED FROM th2

Max1

th2

EXAMINATION BIN VALUE

Fig. 6B

Left peak x 0.10

Right peak x 0.05

Th1

Max1

Th2

Max2

Th3

**Fig. 7**

EP 3 443 907 A1

**Fig. 8**

**Fig. 9**

EP 3 443 907 A1

Fig. 10

Fig. 11

1200

START ~1202

DETERMINE
TRUNK LABEL ~1204

EXTRACT
BONE IN
TRUCK REGION ~1206

DETERMINE
BACKBONE/
PELVIS/
THIGH
BONE LABEL ~1208

CREATE
BOUNDING
RECTANGLE
FOR
EACH SLICE ~1210

EXAMINE
CHANGE AMOUNT
OF
BOUNDING
RECTANGLE ~1212

DETERMINE
LOWER ABDOMEN
START SLICE ~1214

END ~1216

# Fig. 12

Fig. 13

Fig. 14A

Fig. 14B

1500

1502

START

1504

1506

LABELING

1508

DETERMINE
THIGH
BONE LABELS

1510

EXTRACT HOLE

1512

CALCULATE
THIGH BONE
VOLUME

1514

1516

DETERMINE
MAXIMUM VALUE
OF
PELVIC VOLUME

1518

DETERMINE
LOWER EXTREMITY
START SLICE

1520

END

Fig. 15

THIGH BONE LABEL

Fig. 16

1702 1700

START

1704
READ DATA

1706
DETERMINE
HEAD SEARCH
REGION

1708
DETERMINE
PIXEL HAVING
MAXIMUM
PIXEL VALUE

1710
REGION
GROWING

1712
DETERMINE
BLADDER
SEARCH REGION

1714
DETERMINE
PIXEL HAVING
MAXIMUM
PIXEL VALUE

1716
REGION GROWING

1718
STORE
REGION DATA

1720
END

**Fig. 17**

Fig. 18

## INTERNATIONAL SEARCH REPORT

| International application No. |
| --- |
| PCT/JP2017/001460 |

A. CLASSIFICATION OF SUBJECT MATTER
*A61B6/03*(2006.01)i, *G01T1/161*(2006.01)i

According to International Patent Classification (IPC) or to both national classification and IPC

B. FIELDS SEARCHED

Minimum documentation searched (classification system followed by classification symbols)
A61B6/00, G01T1/161, G06T1/00

Documentation searched other than minimum documentation to the extent that such documents are included in the fields searched
Jitsuyo Shinan Koho        1922–1996   Jitsuyo Shinan Toroku Koho   1996–2017
Kokai Jitsuyo Shinan Koho  1971–2017   Toroku Jitsuyo Shinan Koho   1994–2017

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
| --- | --- | --- |
| A | JP 2011-62253 A (Hamamatsu Photonics Kabushiki Kaisha), 31 March 2011 (31.03.2011), entire text; all drawings (Family: none) | 1-24 |
| A | JP 63-24478 A (Pixer), 01 February 1988 (01.02.1988), entire text; all drawings & US 4835712 A          & US 5381518 A & GB 2190570 A          & GB 8708832 A0 & DE 3712639 A          & FR 2597227 A & CA 1258923 A | 1-24 |

☐ Further documents are listed in the continuation of Box C.          ☐ See patent family annex.

*    Special categories of cited documents:
"A"  document defining the general state of the art which is not considered    to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search | Date of mailing of the international search report |
| --- | --- |
| 23 March 2017 (23.03.17) | 04 April 2017 (04.04.17) |

| Name and mailing address of the ISA/ Japan Patent Office 3-4-3,Kasumigaseki,Chiyoda-ku, Tokyo 100-8915,Japan | Authorized officer Telephone No. |
| --- | --- |

Form PCT/ISA/210 (second sheet) (January 2015)

41

**REFERENCES CITED IN THE DESCRIPTION**

**Patent documents cited in the description**

- JP 2013088386 A